# EUROPEAN PATENT APPLICATION

(11) **EP 4 477 240 A1**
(43) Date of publication of application: **18.12.2024**
(21) Application number: 23382599.1
(22) Date of filing: 15.06.2023
(51) Int. Cl.: A61L 31/10, A61L 31/16, A61L 31/06

(54) **A DRUG-ELUTING STENT COMPRISING A BIOCOMPATIBLE THERMOPLASTIC POLYMER LINING AND A DRUG-CONTAINING COATING**

(71) Applicant: Scitech Medical Inc., Doral, FL 33172 (US)
(72) Inventor: MARRA MOREIRA, Alexandre, 04117-110 SAO PAULO (BR); CORDEIRO, Eduardo Jose, 74366-098 GOIÂNIA (BR); NUNES DE SOUSA, Jordana, 74310-260 GOIÂNIA (BR); ALMEIDA FLEURY CURADO, Luciano, 74835-460 GOIÂNIA (BR); DA CUNHA NETO, Melchiades, 74.110-060 GOIÂNIA (BR)
(74) Representative: ZBM Patents - Zea, Barlocci & Markvardsen

(57) **Abstract**

It is provided a drug-eluting stent 10 comprising: an expandable structural scaffold 20 configured to resist radial compression when disposed in a lumen of a patient; a lining 30 arranged only at the central portion 21 of the structural scaffold so that the central portion is covered by the lining and the end portions 22 and 23 are free from the lining; wherein the lining comprises a biocompatible thermoplastic polymer; wherein the proximal and the distal end portions are coated with a drug-containing coating 40 comprising a biodegradable polymer and a Limus drug; and wherein the central portion of the structural scaffold and the lining are free from the Limus drug. It is also provided a process for the preparation of the drug-eluting stent.

## Description

### Technical Field

The present disclosure relates generally to medical devices. More specifically, the present disclosure relates to a drug-eluting stent.

### Background Art

Stents may be deployed in various body lumens for a variety of therapeutic purposes including the treatment of occlusions within the lumens. Stents have a support structure such as a metallic or polymeric structure to provide the strength required to maintain the patency of the vessel in which it is implanted.

One of the drawbacks of vascular stents is the potential for restenosis via the development of a scar tissue (neointima) that forms inside the lumen. Development of a neointima can re-occlude the vessel lumen (restenosis). Substantial improvements have been made in reduction of neointima after stent placement by the use of biocompatible materials, antiinflammatory drug-eluting stents, resorbable stents, and others. An approach consists in providing the stent with a biocompatible polymer (BP) coating to provide a biocompatible surface. Therefore, BP-covered stents such as polytetrafluoroethylene (PTFE)-covered stent may avoid stent dysfunction, including thrombosis, and abnormal pseudointimal proliferation.

Drug-eluting stents (DES) are provided with a surface coating of a polymeric material wherein a therapeutic agent is loaded, what allows the delivery of the therapeutic agent locally to a lumen wall adjacent to the stent. DES are particularly useful in the treatment of atherosclerotic stenosis in arteries and blood vessels, being effective in reducing in-stent neointimal proliferation and, hence, the occurrence of restenosis.

In spite that by the use of BP-covered stents or DES allows significantly reducing restenosis rates compared to bare stenting, restenosis may occur at a higher rate near the outer borders of the stent (stent end stenosis), what is known as the "edge effect". That is, after a stent is implanted, the interior of the artery near its edges may continue to decrease in size, what could eventually cause restenosis and re-block the artery.

Therefore, there is still an interest in reducing the incidence of restenosis after stenting and, more particularly, in reducing edge restenosis.

### Summary of Invention

The present inventors have found that by covering the central portion of the structural scaffold of a stent on the longitudinal direction with a lining of biocompatible thermoplastic polymer, that is leaving the end portions of the structural scaffold without the lining, and coating the two end portions of the structural scaffold with a drug-polymer coating, not only restenosis is avoided, but also stent end stenosis.

By covering the central portion of the scaffolding with the lining of the biocompatible thermoplastic polymer, the formation of a thin endothelial layer on the inside surface of the stent is allowed. In addition, any tissue growth closing the vessel lumen is avoided. This would permit that the stent is more consistent with the surrounding body lumens, thereby reducing turbulent blood flow, decreasing the risk of thrombosis, or the formation of blood clots. In addition, the formation of the endothelial layer can promote the healing at the region of the lumen in need thereof. Hence, in addition to ensuring vessel reperfusion, restenosis is avoided.

At the same time, by coating the end portions of the scaffolding with a drug-containing coating, a controlled release of the drug on the extremes of the stent is allowed and, hence the edge effect (that is, stent end stenosis) is minimized or even avoided.

Advantageously, since the cover of biocompatible thermoplastic polymer in the central portion is not coated with the polymer containing the drug, the effect provided by the former is not altered. Thus, the edge effect is avoided while maintaining the effect of the biocompatible thermoplastic polymer.

In addition, it has been observed that the amount of drug in the polymer coating can be diminished compared to drug-eluting stents completely coated with a drug-containing polymer coating (either in the abluminal surface, in the luminal surface, or both) without having a detrimental effect. Therefore, advantageously, secondary undesirable effects associated to the drug are reduced.

Thus, a first aspect of the present disclosure relates to a drug-eluting stent comprising:
- an expandable structural scaffold configured to resist radial compression when disposed in a lumen of a patient, the structural scaffold comprising a central portion arranged between a proximal end portion and a distal end portion,
- a lining arranged only at the central portion so that the central portion is covered by the lining and the end portions are free from the lining; wherein the lining comprises a biocompatible thermoplastic polymer and, optionally, a fibering agent; and
- wherein the proximal and the distal end portions are coated with a drug-containing coating, the coating comprising a biodegradable polymer and a Limus drug;
wherein the central portion of the structural scaffold and the lining are free from the Limus drug.

A second aspect of the present disclosure relates to a method of manufacturing a drug-eluting stent, the method comprising:
(a) providing an expandable structural scaffold configured to resist radial compression when disposed in a lumen of a patient, the structural scaffold comprising a central portion arranged between a proximal end portion and a distal end portion;
(b) covering only the central portion of the structural scaffold with a lining of a biocompatible thermoplastic polymer so that the end portions are free from the lining, optionally, wherein the structural scaffold has a length from 40 mm to 100 mm and the drug-coated proximal and distal end portions have, independently, a length from 2 mm to 3 mm; and
(c) preparing a coating solution comprising a biodegradable polymer, a Limus drug, a solvent, and, optionally, a second active agent, and applying the coating solution to the proximal and distal end portions of the structural scaffold.

### Brief Description of Drawings

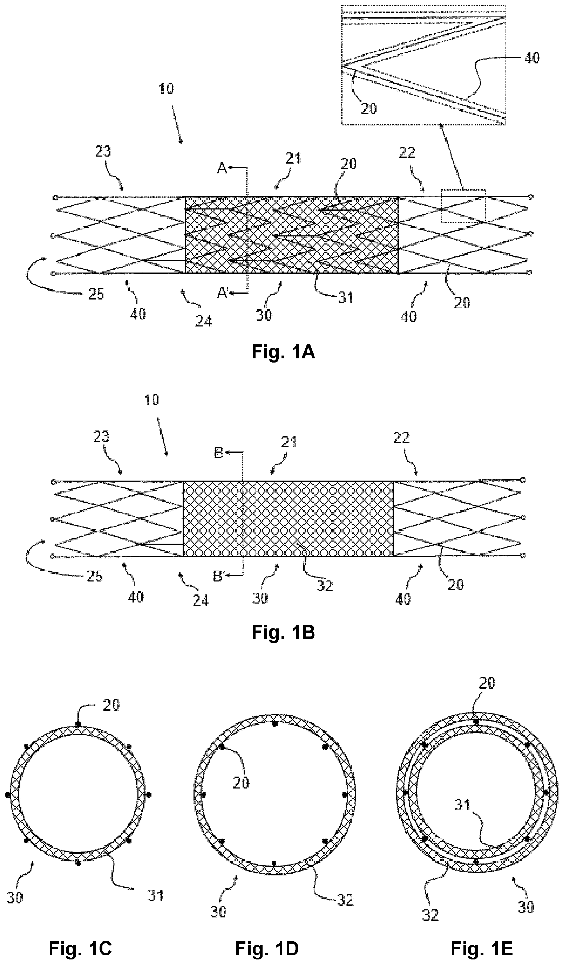

Fig. 1A shows of a drug-eluting stent 10 in accordance with an embodiment of the invention comprising a lining 30 comprising an inner lining layer 31 covering the luminal surface of the central portion of the structural scaffold 20. Fig. 1C represents a cross-sectional view along the line A-A' of the drug-eluting stent 10 of Fig. 1A. Fig. 1B shows of a drug-eluting stent 10 in accordance with another embodiment of the invention comprising a lining 30 comprising an outer lining layer 32 covering the abluminal surface of the central portion of the structural scaffold 20. Fig. 1D represents a cross-sectional view along the line B-B' of the drug-eluting stent 10 of Fig. 1B. Fig. 1E represents a cross-sectional view along the line B-B' of a drug-eluting stent comprising a lining 30 comprising an inner lining layer 31 covering the luminal surface of the central portion of the structural scaffold 20 and an outer lining layer 32 covering the abluminal surface of the central portion of the structural scaffold 20.
Fig. 2 shows a shrink tube positioned on a Solaris^{®} stent before being shrunk.
Fig. 3 shows a shrink tube conforming to a Solaris^{®} stent.
Fig. 4. shows the comparison between minimal vessel diameters measured by IVUS after implantation of a Solaris DE^{®} vascular stent and a Solaris^{®} stent.
Fig. 5. shows the comparison between mean stent areas measured by IVUS after implantation of a Solaris DE^{®} vascular stent and a Solaris^{®} stent.
Fig. 6. shows the comparison between mean stent diameters measured by IVUS after implantation of a Solaris DE^{®} vascular stent and a Solaris^{®} stent.
Fig. 7. shows the comparison between hyperplasia volumes measured by IVUS after implantation of a Solaris DE^{®} vascular stent and a Solaris^{®} stent.
Fig. 8. shows the comparison between Lumen volumes measured by IVUS after implantation of a Solaris DE^{®} vascular stent and a Solaris^{®} stent.
Fig. 9. shows the comparison between neointimal hyperplasia volumes measured through IVUS after implantation of a Solaris DE^{®} vascular stent and a Solaris^{®} stent.
Fig. 10. shows the comparison between the percentages of obstruction by neointimal hyperplasia measured through IVUS after implantation of a Solaris DE^{®} vascular stent and a Solaris^{®} stent.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the term "lumen" refers to the inner open space or cavity of a blood vessel.

The terms, "drug", "therapeutic agent", and "active agent", are herein used interchangeably. A therapeutic agent may be used singly or in combination with other therapeutic agents.

As used herein, the expression "therapeutically effective amount" with respect to a therapeutic agent refers to the amount of a therapeutic agent that, when administered to a human or veterinary patient, is sufficient to prevent the development of, or alleviate to some extent, one or more of the symptoms of the disease or abnormal condition to which is addressed. The particular dose of compound administered according to this invention will of course be determined by the particular circumstances surrounding the case, including the compound administered, the route of administration, the particular condition being treated, the particular circumstances of the individual subject to be treated, and similar considerations.

As used herein, the indefinite articles "a" and "an" are synonymous with "at least one" or "one or more." Unless indicated otherwise, definite articles used herein, such as "the" also include the plural of the noun.

The term "and/or" means that any one of the options to which it relates are possible or the at least two options take place at the same time.

Stents are typically composed of a metallic or polymeric structural scaffold that includes a pattern or network of interconnecting structural elements or struts. The structural scaffold can be provided with a polymeric cover and/or coating.

As used herein, the term "structural scaffold" refers to of scaffolding or stent frame, for example, laser-cut stent frames, polymeric stent frames, wire scaffolding, and so forth. As an instance, a stent may consist of a wire shaped to form scaffolding. The structural scaffold of the stent can be formed in a variety of ways to provide a suitable intraluminal support structure having an outer surface (abluminal surface) for contact with the vessel wall upon implantation, and an inner surface (luminal surface) towards the lumen of the vessel.

As used herein, the term "proximal end" of a stent is defined as the end of the stent closest to the practitioner when the stent is disposed within a deployment device which is being used by the practitioner; and the term "distal end" of a stent is defined as the end opposite the proximal end, along the longitudinal direction of the stent. As used herein, the ends of the stent labelled proximal and distal prior to deployment remain the same regardless of whether the stent is deployed. The "longitudinal direction" of the stent is the direction along the axis of a generally tubular stent.

As used herein, the term "covered" or "lining" refers to a tubular structure of biocompatible thermoplastic polymer covering a surface of the central portion of the structural scaffold. In particular, an "inner lining" is covering the luminal surface of the central portion of the structural scaffold; and an "outer lining" is covering the abluminal surface of the central portion of the structural scaffold.

As mentioned above, a first aspect of the present disclosure relates to a drug-eluting stent comprising an expandable structural scaffold, a lining arranged only at the central portion of the structural scaffold and comprising a biocompatible thermoplastic polymer, wherein the proximal and the distal end portions are coated with a drug-containing coating, the coating comprising a biodegradable polymer and a Limus drug; wherein the central portion of the structural scaffold and the lining are free from the Limus drug.

In a particular embodiment, the lining is drug-free.

In a particular embodiment, optionally in combination with one or more features of the various embodiments described above, the structural scaffold (also known as stent frame) is made of a material selected from a cobalt-chrome alloy (such as MP35N, L605), a Nitinol alloy (a nickel-titanium alloy where the two elements are present in roughly equal atomic percentages, such as Nitinol 55 and Nitinol 60), magnesium alloys, and biodegradable synthetic polymers, such as poly-L-lactic acid (PLLA), poly glycolic acid (PGA), poly (lactide-co-glycolic acid) (PLGA), poly-lactide-co-caprolactone (PLC), and poly-ε-caprolactone (PCL), and hyaluronic acid.

The structural scaffold comprised in the present disclosure can have a variety of diameters and lengths depending on the specific stent application.

In an embodiment, the structural scaffold has a diameter from 5 mm to 10 mm such as of 5, 6, 7, 8,9 and 10 mm, and a length from 40 mm to 100 mm such as or 40, 60, 80, or 100 mm, prior to deployment.

In an example, the drug-eluting stent of the present disclosure can be used in aortic applications. Thus, in another embodiment, optionally in combination with one or more features of the various embodiments described above, the structural scaffold has a diameter from 16 to 45 mm and length from 60 to 200 mm. Other product lines as aortic graft stents have extended sizes.

As mentioned above, the structural scaffold of the drug-eluting stent comprises a luminal surface and an abluminal surface. The lining can be disposed covering the luminal surface of the central portion of the structural scaffold, the abluminal surface of the central portion of the structural scaffold, or both.

Therefore, in another embodiment, optionally in combination with one or more features of the various embodiments described above, the lining comprises an inner lining layer covering the luminal surface of the central portion of the structural scaffold. This can allow the formation of an endothelial layer on the inside surface of the stent, which may be desirable for healing, biocompatibility, prevention of thrombosis, or reduction of the turbulent blood flow within the stent.

In another embodiment, optionally in combination with one or more features of the various embodiments described above, the lining comprises an outer lining layer covering the abluminal surface of the central portion of the structural scaffold. This can permit the healing and the integration of the stent into the body lumen, for instance, by the growth of tissue of the surrounding lumen on the outer lining.

In another embodiment, optionally in combination with one or more features of the various embodiments described above, the lining comprises an inner lining layer covering the luminal surface of the central portion of the structural scaffold, and an outer lining layer covering the abluminal surface of the central portion of the structural scaffold.

In another embodiment, optionally in combination with one or more features of the various embodiments described above, the lining comprises an inner lining layer covering the middle portion of the luminal surface of the structural scaffold, and an outer lining layer covering the middle portion of the abluminal surface of the structural scaffold.

In another embodiment, optionally in combination with one or more features of the various embodiments described above, the biocompatible thermoplastic polymer is selected from the group consisting of polytetrafluoroethylene (PTFE), polyvinylidene fluoride (PVDF), polyurethane, and a mixture thereof. In a particular embodiment, the biocompatible thermoplastic polymer is PTFE, which due to its great tissue compatibility, provided a good matrix for neointimal coverage of the stent surface.

In a particular embodiment, optionally in combination with one or more features of the various embodiments described above, the biocompatible thermoplastic polymer is electrospun PTFE or expanded polytetrafluoroethylene (ePTFE).

In another embodiment, optionally in combination with one or more features of the various embodiments described above, the lining further comprises a fibering agent. Examples of fibering agents are disclosed, for instance, in WO2010083530 In a particular embodiment, the fibering agent is polyethylene oxide (PEO). In a more particular embodiment, the lining comprises PTFE and PEO.

In another embodiment, optionally in combination with one or more features of the various embodiments described above, the biocompatible thermoplastic polymer cover has thickness from 10 to 200 µm, particularly of 30 to 100 µm.

As mentioned above, the drug-containing coating comprises a biodegradable polymer and a Limus drug.

In an embodiment, optionally in combination with one or more features of the various embodiments described above, the a biodegradable polymer is a poly α-hydroxy acid; optionally, wherein the poly α-hydroxy acid is selected from the group consisting of poly-L-lactic acid (PLLA), poly-D-lactic acid (PDLA), poly-lactic acid (PLA; poly-L,D-lactic acid in all their chiral ratios), polyglycolic acid (PGA), poly (lactide-co-glycolic acid) (PLGA; in all the chirality and ratios of the lactic and glycolic groups which form them), and combinations thereof. In a particular embodiment, the biodegradable polymer is PLA, in particular, PLA having a molecular weight (Mw) from 85,000 to 160,000 Da and a viscosity from 0.5 to 1.5 dl/g. In another embodiment, the biodegradable polymer is PLGA (50:50), in particular PLGA (50:50) having a Mw from 30,000 to 60,000 Da and a viscosity from 0.3 to 1.0 dl/g.

In a particular embodiment, the biodegradable polymer is a mixture of PLA and PLGA. In a more particular embodiment, the weight ratio of PLA and PLGA (PLA:PLGA) is from 3:1 to 7:1, particularly from 3:1 to 4:1.

In an embodiment, optionally in combination with one or more features of the various embodiments described above, the drug-containing coating is arranged on the luminal surface, on the abluminal surface, or both, of the proximal and the distal end portions.

In an embodiment, optionally in combination with one or more features of the various embodiments described above, the Limus drug is selected from the group consisting of sirolimus (rapamycin), pimecrolimus, tacrolimus, everolimus, zotarolimus, novolimus, myolimus, temsirolimus, deforolimus, biolimus or combinations thereof.

In a particular embodiment, optionally in combination with one or more features of the various embodiments described above, the Limus drug is sirolimus.

The Limus drug should be incorporated in the device in a therapeutically effective amount, that is, the Limus drug present in the coating should be in an amount that is effective to inhibit restenosis, particularly, stent end stenosis, when the stent is deployed and the drug is delivered to a wall of the vessel that is being treated by the stent. As will be understood by any skilled in the art, the level of a drug that will be therapeutically effective will vary with the specific drug in use, the implant site, the condition to be treated, the composition of the coating including the drug, and other potential factors. A skilled person in the art will know which amount of Limus drug will be a therapeutically effective amount in any circumstance.

In an embodiment, optionally in combination with one or more features of the various embodiments described above, the Limus drug has a concentration from 1 to 10 µg/mm² of coated surface, in particular from 2 to 5 µg/mm² of coated surface.

In another embodiment, optionally in combination with one or more features of the various embodiments described above, the weigh ratio of biodegradable polymer and Limus drug is from 1.7:1 to 3.5:1, particularly from 1:7 to 2:1.

In another embodiment, optionally in combination with one or more features of the various embodiments described above, the drug-containing coating has a thickness of 20 µm.

In another embodiment, optionally in combination with one or more features of the various embodiments described above, the drug-coating coating further comprises a second active agent, optionally, wherein the second active agent is an anticoagulant agent.

In another embodiment, optionally in combination with one or more features of the various embodiments described above, the anticoagulant agent is heparin or a low molecular weight heparin (LMWH).

LMWHs are heparin salts having an average molecular weight of less than 8000 Da and for which at least 60% of all chains have a molecular weight less than 8000 Da. LMWHs can be obtained by various methods of fractionation or depolymerization of polymeric heparin well known in the art.

As mentioned above, the drug-containing coating is applied only on the proximal and distal end portions of structural scaffold, which are not covered with the lining of biocompatible thermoplastic polymer. Therefore, after deployment, the drug will be released from the scaffold struts coated with the drug-containing coating according to a drug release curve, the biodegradable polymer comprised in the drug-containing coating working as drug reservoir. As mentioned above, any person skilled in the art will know how to obtain a drug-containing coating to provide the sought controlled release of Limus drug depending on the circumstances.

The length of the drug-coated proximal end and distal ends should be enough to allow that the drug-containing coating supplies the amount of drug needed to provide the sought effect of minimizing, or even avoiding, stent end stenosis. As an example, the lining can cover from 92.5% to 95.0% of the structural scaffold, either along the luminal surface, the abluminal surface, or both structural scaffold in its longitudinal direction. Hence, the sum of the length of the stent end portions not covered by the lining is from 5.0% to 7.5% of the structural scaffold.

In an embodiment, optionally in combination with one or more features of the various embodiments described above, the drug-coated proximal and distal end portions have, independently, a length from 0.5 to 30 mm, particularly of 5 mm to 30 mm.

In a particular embodiment, optionally in combination with one or more features of the various embodiments described above, the drug-coated proximal end portion, the drug coated distal end portion, or both, have a length from 0.5 to 3 mm, such as of 2 mm, such as in arteriovenous fistulas (AVFs).

In another embodiment, optionally in combination with one or more features of the various embodiments described above, the drug-coated proximal end portion, the drug coated distal end portion, or both, have a length from 10 mm to 30 mm, such as of 15 mm, such as in stents for aortic uses.

Also disclosed herein there is a method of manufacturing a drug-eluting stent as defined herein, the method comprising (a) providing an expandable structural scaffold as defined above; (b) covering only the central portion of the structural scaffold with a lining of a biocompatible thermoplastic polymer such that the proximal and distal end portions are free from the lining; and (c) coating the proximal and distal end portions with a coating solution comprising a biodegradable polymer, a Limus drug, a solvent, and, optionally, a second active agent as defined above.

In the present disclosure, it is noted that when discussing the method for the manufacture of the drug-eluting stent, each one of the embodiments or features defined for the drug-eluting stent can be considered applicable to the method of manufacture, when pertinent, whether or not they are explicitly discussed in the context of that other aspect. Thus, for example, when defining options of the biocompatible thermoplastic polymer, the biodegradable polymer, the Limus drug, the second active agent, or the structural features of the drug-eluting stent (such as disposition of the cover or the coating in the luminal or abluminal surfaces of the central or the end portions, the concentration of drug, the length and diameter or the structural scaffold, the length of the drug-coated end portions, and so on), such options also refer to the method for the manufacture of the drug-eluting stent, when applicable.

In an example, a method for covering the central portion of the structural scaffold with a lining of a biocompatible thermoplastic polymer is electrospinning. Electrospinning is a method for the manufacture of ultra-thin synthetic fibers on charged surfaces. The use of electrospinning for stent coating allows obtaining durable, homogeneous coating while controlling the thickness, density, porosity, and other characteristics of the cover so formed. When a stent is coated by electrospinning, the pores of the cover formed allows the growth of endothelial cells, what reduce the likelihood of negative flow characteristics, thrombosis formation in the luminal surface of the stent, or of inflammatory response on the tissue contacting the abluminal surface of the stent.

Thus, in a particular embodiment, optionally in combination with one or more features of the various embodiments described above, the covering of step (b) is performed by electrospinning.

In an embodiment of the method of the method of the present disclosure, optionally in combination with one or more features of the various embodiments described above, step (b) is performed by electrospinning and the biocompatible thermoplastic polymer is PTFE, namely electrospun PTFE. Electrospun PTFE consists of tubes, mats, or other shapes of PTFE formed from randomly deposited strings of PTFE. Electrospinning can be carried out by depositing a polymer on a surface of the stent in the presence of an electrostatic field. Electrospinning of PTFE is described, for instance, in WO2010083530 and US2016296351.

The drug-containing coating on the surface the proximal and/or distal end portions, which are not covered by the lining of biocompatible thermoplastic polymer, can be applied by any suitable method. For example, the coating can be formed by preparing a coating solution containing the biodegradable polymer, the Limus drug, a solvent, and, optionally, a further active agent, and applying the coating solution by dipping or spraying.

In a particular embodiment of the method of the present disclosure, optionally in combination with one or more features of the various embodiments described above, the coating of the ends of the stent can be performed by applying a solution comprising the Limus drug, the polymer carrier and, optionally, a stabilizer such as ethanol, in a suitable solvent by spray coating. Spray coating can be carried out with a conventional spray coating machine. The solution can be prepared and applied as disclosed in detail in the examples below.

In an embodiment of the method of the present disclosure, optionally in combination with one or more features of the various embodiments described above, the solvent is selected from water, an organic solvent, and mixtures thereof. In another embodiment, the organic solvent is a polar organic solvent. In another embodiment, the organic solvent is selected from the group consisting of ethanol, methanol, hexane, heptane, dichloromethane, chloroform, toluene, benzyl alcohol, and mixtures thereof. In a particular embodiment, the solvent is chloroform. In another embodiment, the solvent is ethanol. In another particular embodiment, the solvent is a mixture of chloroform and ethanol. In still another particular embodiment, the solvent is a mixture of water and ethanol.

In order to avoid the coating of the lining covering a middle portion of the structural scaffold, particularly, when the coating is carried out by spray coating, before carrying the coating of step (c), the lining covering the structural scaffold can be previously protected with a cover that, once finished the coating step (c), will be removed. An example of cover is a shrink tube.

In an example, the cover to protect the lining before coating the proximal and distal end portions can be made of a thermoplastic polymer selected from a polyolefin such as polyethylene including high-density polyethylene (HDPE) and low-density polyethylene (LDPE); fluorinated ethylene propylene; and a Pebax^{®} elastomers (thermoplastic block copolymers made up of rigid polyamide blocks and soft polyether blocks).

With reference to Fig. 1A, one embodiment is shown of a drug-eluting stent 10 in accordance with an embodiment of the invention. Fig. 1C represents a cross-sectional view along the line A-A' of the drug-eluting stent 10 of Fig. 1A. The stent 10 comprises an expandable structural scaffold 20 comprising a central portion 21 arranged between a proximal end portion 22 and a distal end portion 23. The structural scaffold 20 comprises a luminal surface 24 and an abluminal surface 25. The luminal surface 24 defines the inner surface of the structural scaffold, and the abluminal surface 25 defines the outer surface of the structural scaffold 20. A lining 30 comprising a biocompatible thermoplastic polymer is arranged covering only the central portion 21 so that the end portions 22 and 23 are free from the lining 30. The lining 30 of these figures comprises an inner lining layer 31 covering the luminal surface of the central portion of the structural scaffold 20. The central portion 21 of the structural scaffold 20 is thus covering the inner lining layer 31. The proximal end portion 22 and the distal end portion 23 are coated with a drug-containing coating 40 comprising a biodegradable polymer and a Limus drug. The central portion 21 of the structural scaffold 20 and the lining 30 are free from Limus drug. The drug-containing coating 40 can be arranged on the luminal surface, on the abluminal surface, or both, of the proximal and the distal end portions.

In another example the lining 30 comprises an outer lining layer 32 covering the abluminal surface of the central portion of the structural scaffold 20 as shown in Fig. 1B. A cross-sectional view of the drug-eluting stent along the line B-B' is shown in Fig. 1D. As in Fig. 1A and 1C, the proximal end portion 22 and the distal end portion 23 of Fig. 1B and 1D are not covered by the lining 30.

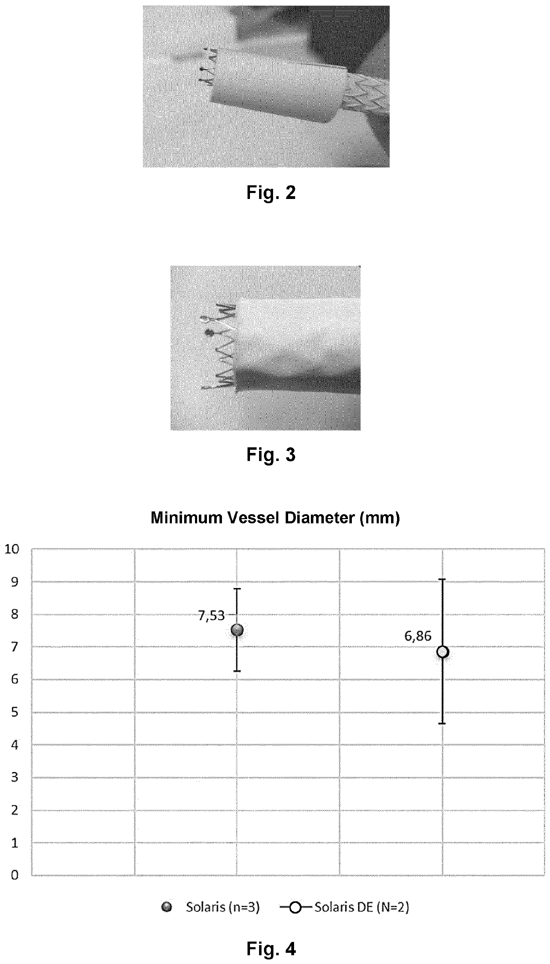

Fig. 2E shows another example of a cross-sectional view of the central portion 21 of the structural scaffold 20. In this example, the lining comprises an inner lining layer 31 covering the luminal surface of the central portion of the structural scaffold 20 and an outer lining layer 32 covering the abluminal surface of the central portion of the structural scaffold 20.

Stents may be deployed in various body lumens for a variety of purposes, for example, in the central venous system for a variety of therapeutic purposes including the treatment of occlusions within the lumens of that system. It will be appreciated that the current disclosure may be applicable to stents designed for the peripheral arterial disease (PAD) abdominal aortic aneurism (AAA), arteriovenous fistula and so on.

The stent of the present disclosure can be used for treating a disorder in a blood vessel, in particular to dilate a constricted blood vessel, and more particularly, to treat or prevent stenosis or restenosis in the blood vessel, even more particularly, to prevent stent edge stenosis. In an embodiment, the stent of the present disclosure can be used in the treatment of arteriovenous fistulas, obstructive diseases, aneurisms, vessel dissections.

Throughout the description and claims the word "comprise" and variations of the word, are not intended to exclude other technical features, additives, components, or steps. Furthermore, the word "comprise" encompasses the case of "consisting of".

The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### EXAMPLE 1 - Method of obtaining a Solaris drug-eluting (Solaris DE) stent

### Preparation of the coating solution

The Solaris^{®} is a flexible, self-expanding stent, comprised of a thin, multi-direction, durable electrospinning PTFE membrane encapsulating a Nitinol stent structure. The stent is laser cut from a metal tube of Nickel-titanium alloy (Nitinol) to form a metallic scaffold which is subsequently covered with polytetrafluorethylene (PTFE) layers. At the ends of the structural scaffold of the stent there are radiopaque markers of tantalum to delimit the stent limits under fluoroscopy. The delivery system is "Pull Back" type and OTW (Over the Wire) composed of concentric tubes that moves to allow external tube retraction on the stent implantation procedure.

Solaris^{®} stents of 5, 6, 7, 8 ,9 and 10 mm of diameter and a length of 40 to 100 mm, having uncovered end portions of 2 to 3 mm were used as starting material for the preparation of a Solaris DE stent.

At the proximal and distal ends of the stent not covered with PTFE, a Sirolimus coating was applied as explained below in order to obtain a Solaris DE stent. The coating formulation contained sirolimus drug as an active ingredient a biodegradable polymer matrix of PLA and PLGA.

Drug-coating solutions containing PLA, PLGA, and rapamycin in chloroform were prepared according to the amounts shown in Table 1 below.

**Table 1: Mass of the raw materials corresponding to each solution volume**

| **Solution Volume** | **PLA** | **PLGA** | **Rapamycin** |
|---|---|---|---|
| 50 mL | 0.125-0.129 g | 0.035-0.039 g | 0.092-0.096 g |
| 100 mL | 0.253-0.257 g | 0.073-0.077 g | 0.187-0.191 g |
| 250 mL | 0.635-0.639 g | 0.185-0.189 g | 0.470-0.474 g |
| 350 mL | 0.891-0.896 g | 0.261-0.266 g | 0.660-0.664 g |
| 500 mL | 1.273-1.277 g | 0.373-0.377 g | 0.943-0.947 g |
| 750 mL | 1.911-1.913 g | 0.561-0.565 g | 1.416-1.420 g |
| 1,000 mL | 2.548-2.552 g | 0.748-0.752 g | 1.888-1.892 g |
| 1,500 mL | 3.823-3.827 g | 1.123-1.127 g | 2.833-2.837 g |
| 2,000 mL | 5.098-5.102 g | 1.498-1.502 g | 3.778-3.782 g |

Previously to being coated, the stents were washed with isopropyl alcohol.

Shrink tubes of 20 mm ± 5 mm, made of a HDPE, and having a length were used as protectors to avoid the drug-containing coating covers the PTFE-cover. A cut was mad on a side of each tube to ease the insertion of the stent inside.

Shrink tubes were positioned at the ends of the stent, so that the tube overlapped the PTFE coating and the cut was pointed towards the center of the stent (Fig. 2).

The assembly of the stent with the shrink tube was positioned in front of a hot air blowing machine, set at a temperature of at 130 °C ± 10 °C, and the assembly was rotated while flowing hot are at a minimum value to promote conformation (Fig. 3.

The conformation was made to allow a minimum movement of the shrink tube if necessary, and to protect the PTFE cover while leaving the structural scaffold end portions free.

### Drug coating of the stent end portions

Coating of the ends of the stents was carried out by spray coating (Coating Machine 1.0-Scitech Medical).

The process was carried out at a relative humidity of 60% to 65% and a temperature from 25 °C to 30 °C. The airbrush flow was calibrated to 0.5 ml in 35 s ± 2s.

To cover every end, the stent was fixed in a mandrel. For each end or the stent, the volumes of coating solutions shown in Table 2 were introduced in the airbrush container.

**Table 2 - Volume of coating solution**

| **Stent** | **Coating Volume at each** | **Total volume of stent coating** |
|---|---|---|
| 8 Peaks (D = 5 mm) | 4 ml* | 8 ml |
| 9 Peaks (D = 6 / 7 / 8 mm) | 5 ml* | 10 ml |
| 10 Peaks (D = 9 mm) | 2 ml* | 4 ml |

| | | |
|---|---|---|
| *Other volumes can be used according to coating machine parameters; the variation can reach up to 200%. | | |

The process was repeated for every end of the stent, by repositioning the stent after coating one of the ends so that the uncoated end was in front of the airbrush. The ends of the stent were thus coated with a whitish and uniform coating. Then, the shrink tubes were removed by tearing them by the cut ends, approximately 10 minutes after the coating process is finished.

The 5 mm, 6 mm and 7 mm diameter stents were packed in cryogenic tubes, and the 8 mm and 9 mm diameter stents in amber glass tubes.

The obtained Solaris DE were stored in Tyvek and then in a desiccator protected from light and moisture. A vacuum of at least 300 mmHg was also applied.

### EXAMPLE 2 and COMPARATIVE EXAMPLE 1. Pre-clinical study of Solaris DE vascular stent graft

The study aimed to evaluate the mechanical and biological performance of the Solaris DE endoprosthesis of the invention in the peripheral arterial vasculature of the swine model compared to Solaris^{®} (PTFE-covered stent without sirolimus commercialized by Scitech Medical S.A.). Vascular response was determined in vivo by intravascular imaging and related histopathological evaluation.

The following specific objectives were investigated:
- safety assessment of the Solaris DE vascular stent graft implanted in peripheral arteries in a porcine model;
- comparison of luminal diameter loss between the 2 types of implanted stents, i.e. the Solaris stent (drug free) and Solaris DE stent (drug based);
- angiographic patency and percent diameter of the stenosis comparison between the Solaris DE and its version without sirolimus (Solaris^{®});
- neointimal formation and comparison of the mean percentage of the stenosis area between test and control by intravascular imaging (IVUS);
- degree of thrombogenicity.

### Materials

| | |
|---|---|
| Number of animals: | 12 |
| Species: | Porcine (Sus scrofa domestica) |
| Breed or strain: | Large White |
| Weight range (at implant): | 25 - 40 kg |
| Age range (at implant): | Juvenile |
| Sex: | Female or castrated male |
| Diet: | Regular growing diet (Pellets) |
| Source of Animals: | Animal Institute, Grodziec Śl 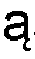 skie |
| Method of identification: | Ear tag |

Animals included into the study had similar weights and ages. Pigs were acclimated to the facility seven days before the beginning of the study. After implantation, follow up was performed at 30 and 90 days (n=6/time-point). A total number of 12 animals were used for the study.

### Sampling

Test (Example) and control (Comparative Example) stents characteristics are described in Tables 3 and 4, respectively.

**Table 3**

| **Name** | **Solaris^{®} DE** |
|---|---|
| Description | Sirolimus-eluting PTFE-coated of the invention |
| Manufacturer | Scitech Medical S.A. |
| Sizes used in study | Sizes: 5 and 6 mm x 40 mm |
| Sterilization | Ethylene oxide |
| Information about packing | Packed in individual units with delivery system. |

**Table 4**

| **Name** | **Solaris^{®}** |
|---|---|
| Description | PTFE-coated stent without sirolimus |
| Manufacturer | Scitech Medical S.A. |
| Sizes used in study | Sizes: 5 and 6 mm x 40 mm |
| Sterilization | Ethylene oxide |
| Information about packing | Packed in individual units with delivery system. |

### Acceptance Criteria

In this study, Solaris DE endograft was evaluated for some endpoints and compared to the Solaris vascular stent graft product.

The primary endpoint is lumen area and % stenosis. Both parameters are inversely proportional.

Besides, the following parameters of stent arteries were also assessed:
- cell growth and cell types;
- inflammation (degree and cell types);
- formation of seroma/voids between the graft and the adventitia;
- assessment of adventitia (necrosis, fibrosis, inflammation, others).

Both, the Solaris DE endograft and the Solaris vascular stent graft were be identified as equivalent to the competing product on the primary endpoint.

As will be seen below, the results showed that Solaris DE was more efficient than Solaris in preventing the treated vessel restenosis.

### Study data

Pigs included in the study had similar weights (40-43 Kg) and ages. They were acclimated to the facility three days before the start of the study.

The animals were submitted to a 12-hour food fast and a 6-hour water fast, and were anesthetized according to a standard procedure before stent implantation.

To start the implant, an 8F or 9F sheath was used (depending on the caliber of the stent delivery system) inserted into the femoral artery. 100-200 IU/kg of heparin was administered intravenously prior to selective catheterization for stent implantation.

Initially, angiographies of the aorta and iliac arteries were performed with an appropriately sized multipurpose catheter, positioned in the topography of the distal aorta, just below the renal arteries. Then, under fluoroscopy, with the aid of a guide wire, the right and left common iliac arteries received one stent each (according to Table 4), respecting the maximum mean diameter of 0.5 mm to 1 mm in relation to the artery. The catheter was removed, and a post-implant image acquired. Finally, the catheter and guidewire were removed, and hemostasis was performed by suturing the access site. During the entire procedure, the animals remained in the supine position. Implant matrix used is shown in Table 5.

**Table 5**

| Animal No. | **Sex** | Right iliac artery | Left iliac artery |
|---|---|---|---|
| 1 | F | Solaris 5 mm x 40 mm | Solaris DE 5 mm x 40 mm |
| 2 | M | Solaris DE 5 mm x 40 mm | Solaris 5 mm x 40 mm |
| 3 | F | Solaris 5 mm x 40 mm | Solaris DE 5 mm x 40 mm |
| 4 | M | Solaris DE 6 mm x 40 mm | Solaris 6 mm x 40 mm |
| 5 | F | Solaris 5 mm x 40 mm | Solaris DE 5 mm x 40 mm |
| 6 | F | Solaris DE 6 mm x 40 mm | Solaris 6 mm x 40 mm |
| 7 | F | Solaris 6 mm x 40 mm | Solaris DE 6 mm x 40 mm |

Each animal received a Solaris DE^{®} vascular stent and a Solaris^{®} stent in each iliac artery. Angiography was performed at four times: pre- and post-stent, after 28 days in all animals and after 60 days (subgroup of 4 animals).

Angiography will be performed shortly after stent implantation to assess the correct position of the stents. After angiographic imaging, the animals evaluated after 60 days were also subjected to IVUS evaluation of the stent segments to determine the occurrence of neointimal formation. The acquired images were analyzed using appropriate measurement software and provided measurements for luminal areas, stent areas, areas of neointimal hyperplasia and % neointimal hyperplasia.

The vascular access site was observed and evaluated once daily for a minimum of 7 days for signs of infection or inflammation.

After delivery of the test article and control device, the delivery systems were evaluated for thrombogenicity. Thrombus levels were graded using an FDA recognized thrombogenicity scale: 0 = no significant thrombus; 1 = minimal thrombus in a single location; 2 = minimal thrombosis in multiple locations; 3 = significant thrombus in half of the inserted catheter segment; 4 = significant thrombus > 50%; and 5 = 100% circumference and length of catheter covered with thrombus. Both articles were rated 0 = no significant thrombus.

Qualitative vascular analysis was performed at four times: pre-and post-stent, after 28 days in all animals and after 60 days (subgroup of 4 animals). Technical success was defined as adequate stent implantation, that is, stent implantation in the predetermined location of the artery and less than 30% recoil after stent implantation was achieved in 100% of cases. There was no change in stent integrity (i.e., stent deformation) or migration on the day of implantation or at the 28- and 60-day follow-ups. No fracture was observed either. Through visual analysis no difference in performance between the test and control device could be identified.

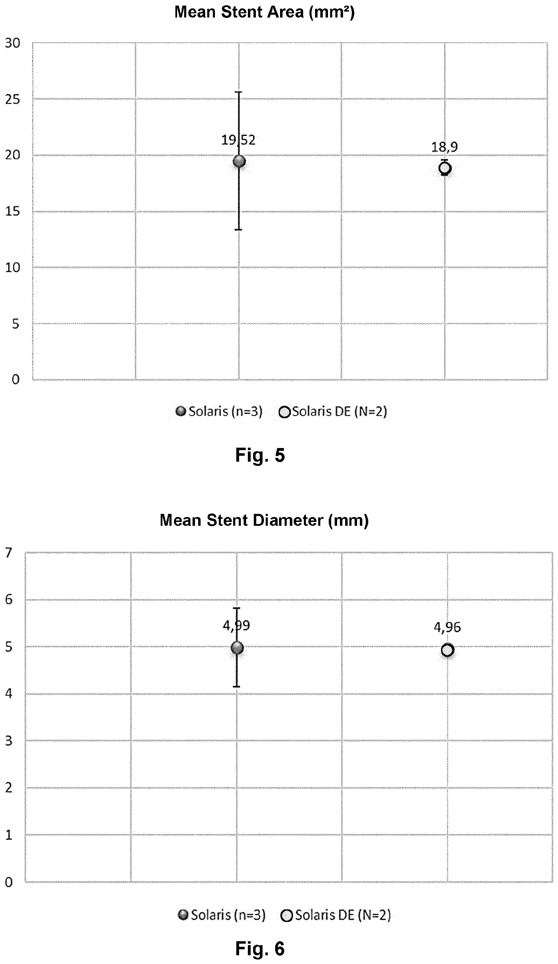

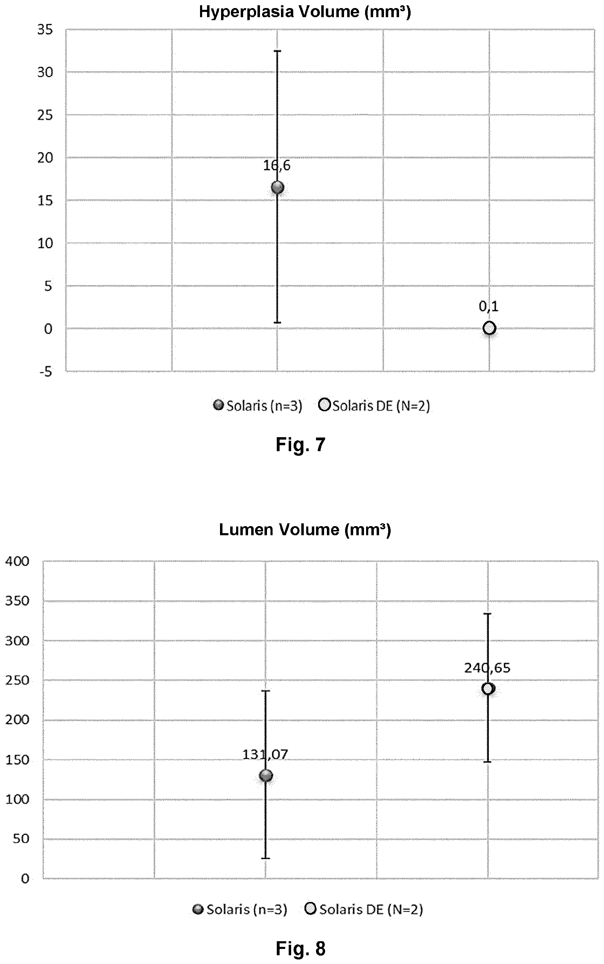

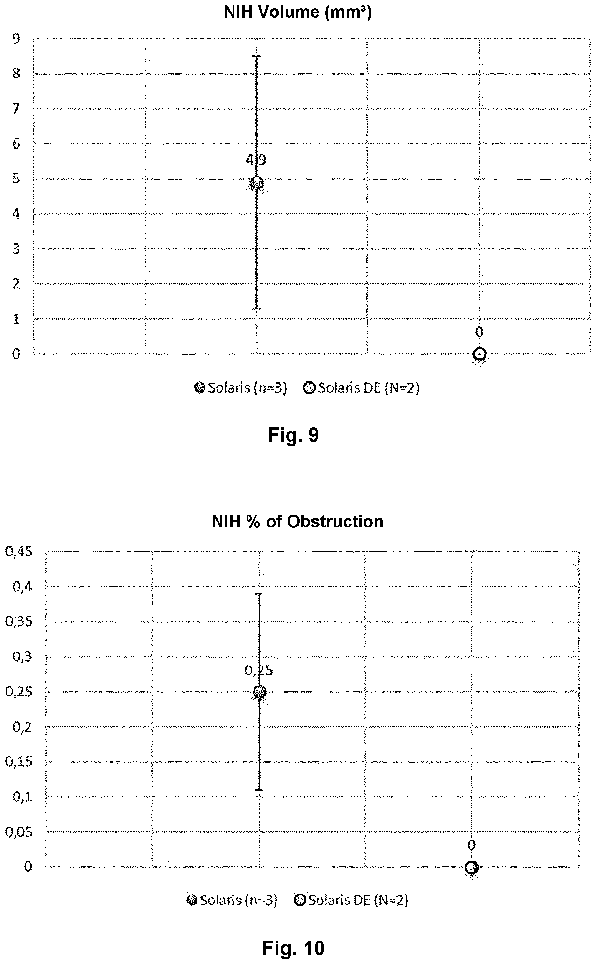

IVUS analysis was performed in 3 animals at 60 days post implantation and the results are shown in Tables 6-8 (see Figures 4 to 10).

**Table 6**

| **VARIABLE** | **Solaris (n=3)** | **Solaris DE (N=2)** | **Difference [Cl 95%]*** | **P*** |
|---|---|---|---|---|
| **Mean Lumen Area (mm²)** | | | | |
| Mean ± Standard Deviation | 14.64 ± 5.34 | 18.90 ± 0.64 | -4.26 [ -16.96; 8.44] | 0.364 |
| Median [25%, 75%] | 14.54 [11.95; 17.29] | 18.90 [18.68; 19.13] | | |
| Min; Max | 9.36; 20.03 | 18.45; 19.35 | | |

| Mean Lumen Diameter (mm) | | | | |
|---|---|---|---|---|
| Mean ± Standard Deviation | 4.32 ± 0.80 | 4.96 ± 0.08 | -0.64 [ -2.53; 1.26] | 0.363 |
| Median [25%, 75%] Min; Max | 4.36 [3.94; 4.73] | 4.96 [4.93; 4.99] | | |
| Min; Max | 3.51; 5.10 | 4.90; 5.02 | | |

| Min Diameter Lumen (mm) | | | | |
|---|---|---|---|---|
| Mean ± Standard Deviation | 3.94 ± 0.60 | 4.55 ± 0.10 | -0.61 [ -2.05; 0.82] | 0.268 |
| Median [25%, 75%] Min; Max | 3.99 [3.65; 4.25] | 4.55 [4.52; 4.59] | | |
| Min; Max | 3.31; 4.51 | 4.48; 4.62 | | |

| **Mean Vessel Area (mm²)** | | | | |
|---|---|---|---|---|
| Mean ± Standard Deviation | 52.64 ± 12.10 | 42.61 ± 25.88 | 10.03 [ -42.01; 62.07] | 0.583 |
| Median [25%. 75%] Min; Max | 48.98 [45.89; 57.57] | 42.61 [33.46; 51.76] | | |
| Min; Max | 42.79; 66.15 | 24.31; 60.91 | | |

| Mean Vessel Diameter (mm) | | | | |
|---|---|---|---|---|
| Mean ± Standard Deviation | 8.19 ± 0.94 | 7.24 ± 2.29 | 0.95 [ -3.49; 5.39] | 0.544 |
| Median [25%. 75%] Min; Max | 7.95 [7.68; 8.59] | 7.24 [6.43; 8.05] | | |
| Min; Max | 7.40; 9.23 | 5.62; 8.86 | | |

**Table 7**

| **VARIABLE** | **Solaris (n=3)** | **Solaris DE (N=2)** | **Difference [Cl 95%]*** | P* |
|---|---|---|---|---|
| **Minimum Vessel Diameter (mm)** | | | | |
| Mean ± Standard Deviation | 7.53 ± 1.26 | 6.86 ± 2.21 | 0.68 [ -4.09; 5.44] | 0.683 |
| Median [25%, 75%] Min; Max | 7.49 [6.89; 8.15] | 6.86 [6.07; 7.64] | | |
| Min; Max | 6.29; 8.81 | 5.29; 8.42 | | |

| **Mean Stent Area (mm²)** | | | | |
|---|---|---|---|---|
| Mean ± Standard Deviation | 19.52 ± 6.12 | 18.90 ± 0.64 | 0.62 [-13.94; 15.17] | 0.901 |
| Median [25%, 75%] Min; Max | 22.49 [17.49; 23.04] | 18.90 [18.68; 19.13] | | |
| Min; Max | 12.48; 23.58 | 18.45; 19.35 | | |

| **Mean Stent Diameter (mm)** | | | | |
|---|---|---|---|---|
| Mean ± Standard Deviation | 4.99 ± 0.83 | 4.96 ± 0.08 | 0.03 [ -1.93; 1.99] | 0.964 |
| Median [25%, 75%] Min; Max | 5.40 [4.72; 5.47] | 4.96 [4.93; 4.99] | | |
| Min; Max | 4.04; 5.53 | 4.90; 5.02 | | |

| Min Diameter of Stent (mm) | | | | |
|---|---|---|---|---|
| Mean ± Standard Deviation | 4.60 ± 0.78 | 4.87 ± 0.54 | -0.26 [ -2.32; 1.80] | 0.713 |
| Median [25%, 75%] Min; Max | 4.86 [4.30; 5.04] | 4.87 [4.67; 5.06] | | |
| Min; Max | 3.73; 5.22 | 4.48; 5.25 | | |

| **Hyperplasia Volume (mm³)** | | | | |
|---|---|---|---|---|
| Mean ± Standard Deviation | 16.60 ± 15.88 | 0.10 ± 0.14 | 16.50 [-21.17; 54.17] | 0.258 |
| Median [25%, 75%] Min; Max | 12.60 [7.85; 23.35] | 0.10 [0.05; 0.15] | | |
| Min; Max | 3.10; 34.10 | 0.00; 0.20 | | |

| **Obstruction Volume (mm³)** | | | | |
|---|---|---|---|---|
| Mean ± Standard Deviation | 0.10 ± 0.06 | 0.00 ± 0.00 | | |
| Median [25%, 75%] Min; Max | 0.12 [0.08; 0.14] | 0.00 [0.00; 0.00] | | |
| Min; Max | 0.04; 0.15 | 0.00; 0.00 | | |

**Table 8**

| **VARIABLE** | **Solaris (n=3)** | **Solaris DE (N=2)** | **Difference [Cl 95%]*** | **P*** |
|---|---|---|---|---|
| **Vessel Volume (mm³)** | | | | |
| Mean ± Standard Deviation | 375.07 ± 305.71 | 542.20 ± 28.43 | -167.13 [-893.87; 559.60] | 0.517 |
| Median [25%, 75%] Min; Max | 256.00 [201.40; 489.20] | 542.20 [532.15; 552.25] | | |
| Min; Max | 146.80; 722.40 | 522.10; 562.30 | | |

| **Stent Volume (mm³)** | | | | |
|---|---|---|---|---|
| Mean ± Standard Deviation | 147.63 ± 121.09 | 240.65 ± 93.27 | -93.02 [ -420.09; 234.06] | 0.432 |
| Median [25%, 75%] Min; Max | 83.60 [77.80; 185.45] | 240.65 [207.68; 273.63] | | |
| Min; Max | 72.00; 287.30 | 174.70; 306.60 | | |

| **Lumen Volume (mm³)** | | | | |
|---|---|---|---|---|
| Mean ± Standard Deviation | 131.07 ± 105.69 | 240.65 ± 93.27 | -109.58 [ -405.09; 185.92] | 0.323 |
| Median [25%, 75%] Min; Max | 71.10 [70.05; 162.10] | 240.65 [207.68; 273.63] | | |
| Min; Max | 69.00; 253.10 | 174.70; 306.60 | | |

| **NIH Volume (mm³)** | | | | |
|---|---|---|---|---|
| Mean ± Standard Deviation | 4.90 ± 3.60 | 0.00 ± 0.00 | | |
| Median [25%, 75%] Min; Max | 3.16 [2.84; 6.10] | 0.00 [0.00; 0.00] | | |
| Min; Max | 2.51; 9.04 | 0.00; 0.00 | | |

| **NIH % of Obstruction** | | | | |
|---|---|---|---|---|
| Mean ± Standard Deviation | 0.25 ± 0.14 | 0.00 ± 0.00 | | |
| Median [25%, 75%] Min; Max | 0.25 [0.18; 0.32] | 0.00 [0.00; 0.00] | | |
| Min; Max | 0.11; 0.38 | 0.00; 0.00 | | |

| | | | | |
|---|---|---|---|---|
| *NIH:* neointimal hyperplasia | | | | |

The tested SOLARIS DE product fulfilled all expectations of efficiency in this pre-clinical trial without raising any safety concerns. The results indicate that the test group showed an adequate safety profile in the preclinical mode, and that there is a tendency for the stent of the invention (Solaris DE stent) to have less hyperplasia that the control stent (Solaris non drug-eluting stent).

### COMPARATIVE EXAMPLES 2 to 4

Inspiron^{®} DES (commercialized by Scitech Medical S.A.) is a stent made of a platform material of CoCr and thin struts (75 µm) and an abluminal coating composed of a mixture of PLA and PLGA polymers and a low Sirolimus dosage.

Thrombosis with sirolimus-polymer was compared with controls with no coating and with only polymer coating with no drugs. Thrombosis was assessed clinically (sudden death of the animal after the procedure with autopsy showed thrombotic occlusion of the stent), by angiographic or histological analysis. The presence medial necrosis with aneurysm formation of polymer-coated stent with sirolimus compared with controls with no coating and polymer coating only with no drugs was determined by histology or angiography (aneurysm, defined as dilation 150% of the vascular diameter of reference). The occurrence of exacerbated neointimal formation of polymer-coated stent with sirolimus compared with controls with no coating and polymer coating only with no drugs was evaluated by histology (neointimal area by histomorphometry) or angiography (late lumen loss). The primary efficacy endpoint was the reduction of neointimal proliferation of polymer-coated stent with sirolimus compared with no drugs.

### Methodology

Fourteen juvenile pork of Large-White (22-30kg) pretreated at least one day before the procedure with 75 mg clopidogrel and 100 mg of aspirin and kept for 28 days and until the end of the study respectively. As a prophylactic measure was an administered intramuscular antibiotic. The animals received premedication with bromazepam and anesthesia induced by thiopental (12 mg/kg), followed by endotracheal intubation, inhalation anesthesia with isoflurane. Intravenous heparin (10,000 IU) was administrated before coronary manipulation.

The animals underwent implantation of three types of coronary stents.
- Comparative example 2: bare metal stent (BMS) - Stent brand Cronus^{®} - Cobalt chromium stent Scitech. - without coating;
- Comparative example 3: stent covered only with polymer;
- Comparative example 4: stent covered with polymer and sirolimus - Inspiron^{®} DES, i.e. Cronus^{®} stent coated with sirolimus in a polymer carrier at the concentration of 1.4 µg/mm², polymer carrier: biodegradable polymer blend of PLLA and PLGA.

Each animal received three stents implanted in right coronary, left descending anterior and circumflex arteries. The animals were followed for 1 month. After one month, all animals underwent repeat coronary angiography control and sacrificed immediately after. The specimens were fixed by perfusion in 10% formalin and analyzed by optical microscopy.

The basal and control angiograms were analyzed quantitatively to assess the presence of stent migration, intraluminal negative image or involvement of the lateral branch.

Quantitative angiographic analysis was performed using the CASS II system (Pie Medical, Maastricht, Netherlands). Were analyzed the in-stent segment, and 5 mm proximal and distal to the stent. The following parameters were measured at baseline and control angiograms in each segment (in-stent, proximal edge and distal edge), minimum luminal diameter (MLD), reference diameter (RD), luminal stenosis in diameter (ELD). Based on these measures the angiographic late loss, defined as control DLM - DLM basal, was calculated. Aneurysmal dilatation was defined as an increase in diameter in the treated area 150 % of reference diameter.

Immediately after sacrifice, the hearts were explanted and perfused for 30 minutes under pressure (~80 mm Hg) through the aortic root with 10% formalin. The arterial segments and the adjacent tissue dissected form the epicardial surface and dried in progressive ethanol solutions for inclusion in plastic resin. The stent was cut into segments of 5 mm. For each segment was obtained with 1-10-micron sections mm thick, stained for histological analysis with hematoxylin-eosin, van Gieson dye and Masson's trichrome.

All sections were measured with the help of software histomorphometric analysis. Cutting greater stenosis was considered for the final tabulation of the data ("worst-view approach). Sectional areas were measured internal elastic lamina (LEI) and the vascular lumen. The neointima area was calculated as 100X (LAW - light area) /LAW. The thickness of the neointima was measured perpendicular to the light from each stent strut.

The severity of vascular injury caused by stent implantation was measured according to the following criteria:

| **Score** | **Description** |
|---|---|
| 0 | Internal elastic lamina intact, endothelium typically denuded, media compressed but not lacerated |
| 1 | Internal elastic lamina lacerated; media compressed but not lacerated |
| 2 | Internal elastic lamina lacerated; media visibly lacerated; external elastic lamina compressed but not lacerated |
| 3 | External elastic lamina lacerated, typically large lacerations of the middle one extending to the external elastic lamina, stent struts sometimes present in the adventitia. |

The animals gained weight over a period of four weeks. Neointimal reduction in angiographic and intravascular ultrasound analysis are shown in Table 9. The results show that the animals who had restenosis were the ones of comparative example 2 (BMS) and comparative example 3 (covered only with polymer polymer).

**Table 9**

| Stent / Data | Late loss (mm) | Neointima area (mm²) | Neointima obstruction (%) |
|---|---|---|---|
| Comp. Ex. 2 (BMS) | 1.03 ± 0.61 | 2.84 ± 2.23 | 39.96 ± 29.00 |
| Comp. Ex. 3 (polymer) | 1.12 ± 0.54 | 2.70 ± 1.40 | 38.53 ± 21.00 |
| Comp. Ex. 4 (Inspiron^{®}) | 0.78 ± 0.31 | 1.30 ± 1.18 | 20.28 ± 16.00 |

General data of studied animals is shown in Table 10 below.

**Table 10**

| **Nr. Animal** | **Gender** | **Artery** | **Stent** | **Size** | **Angle** | **Initial weight** | **Final weight** | **Visual observations of the restudy** |
|---|---|---|---|---|---|---|---|---|
| 37 | Male | CD | BMS | 3,0x19mm | 45° OAE | 31kg | 28,5kg | Excellent aspect ultrassongrafic |
| | | CX | POLYMER | 3,0x19mm | | | | Little neointima (position 8 hours) |
| | | DA | DES | 3,0x19mm | | | | Neointima sharper (initiation of restenosis) |
| 38 | Female | CD | BMS | 3,0x19mm | 60° OAE | 30kg | 27,5kg | Restenosis |
| | | CX | POLYMER | 3,5x19mm | | | | Intimal moderate hyperplasia |
| | | DA | POLYMER | 3,0x19mm | | | | Intimal moderate hyperplasia |
| 39 | Male | CD | POLYMER | 3,0x19mm | 50° OAE | 30kg | 27,3kg | Restenosis (hyperplasia mild to moderate) |
| | | CX | DES | 3,5x19mm | | | | Excellent |
| | | DA | POLYMER | 3,0x19mm | | | | Mild Hyperplasia |
| 40 | Male | CD | DES | 3,0x19mm | 45° OAE | 31,5kg | 31,2kg | Excellent result |
| | | CX | BMS | 3,0x19mm | | | | Hyperplasia moderate |
| | | DA | POLYMER | 3,0x19mm | | | | Restenosis moderate |
| 41 | Male | CD | DES | 3,0x19mm | 45° OAE | 28,5kg | 28,5kg | Mild Hyperplasia |
| | | CX | POLYMER | POLYMER | | | | Mild Hyperplasia |
| | | DA | POLYMER | 3,0x19mm | | | | Intense Hyperplasia |
| 42 | Male | CD | POLYMER | 3,0x19mm | 45° OAE | 30kg | 32kg | Moderate Hyperplasia |
| | | CX | DES | 3,0x19mm | | | | Mild Hyperplasia |
| | | DA | BMS | 3,5x19mm | | | | Restenosis accentuated |
| 43 | Male | CD | DES | 3,0x19mm | 45° OAE | 30kg | 30,5kg | Mild Hyperplasia |
| | | CX | BMS | 3,0x19mm | | | | Intense Restenosis |
| | | DA | POLYMER | 3,0x19mm | | | | Moderated Restenosis |
| 44 | Male | CD | POLYMER | 3,0x19mm | 45° OAE | 26kg | 27,5kg | Mild Hyperplasia |
| | | CX | POLYMER | 2,5X19mm | | | | Mild Hyperplasia |
| | | DA | BMS | 2,5X19mm | | | | Mild Hyperplasia |
| 45 | Male | CD | DES | 2,5X19mm | 45° OAE | 31kg | 32kg | Mild Hyperplasia |
| | | CX | POLYMER | 3,0x19mm | | | | Mild Hyperplasia |
| | | DA | DES | 2,5X19mm | | | | Mild hyperplasia with restenosis at the proximal edge |
| 46 | Female | CD | POLYMER | 3,0x19mm | 45° OAE | 27,5kg | | Probable death of the animal in infarction CD |
| | | CX | BMS | 3,5x19mm | | | | |
| | | DA | DES | 2,5X19mm | | | | |
| 47 | Female | CD | DES | 3,0x19mm | 45° OAE | 26,5kg | 28,3 | Mild hyperplasia |
| | | CX | DES | 3,5x19mm | 60° OAE | | | Mild hyperplasia at the distal region |
| | | DA | DES | 3,0x19mm | 60° OAE | | | Excellent |
| 48 | Male | CD | POLYMER | 3,5x19mm | 45° OAE | 27,5kg | 26 | Discreet hyperplasia, moderate hyperplasia at the proximal edge |
| | | CX | DES | 3,0x19mm | | | | Mild Hyperplasia |
| | | DA | POLYMER | 3,5x19mm | | | | Hyperplasia accentuated |
| 49 | Male | CD | POLYMER | 3,5x19mm | 45° OAE | 27kg | 26,3 | Hyperplasia moderate |
| | | CX | DES | 3,0x19mm | | | | Mild hyperplasia distal |

| | | DA | DES | 3,0x19mm | | | | Hyperplasia at the proximal edge |
|---|---|---|---|---|---|---|---|---|
| 50 | Female | CD | DES | 3,0x19mm | 45° OAE | 26,5kg | 31,5 | Mild Hyperplasia |
| | | CX | POLYMER | 3,5x19mm | | | | Mild Hyperplasia |
| | | DA | BMS | 3,0x19mm | | | | Mild Hyperplasia |
| 51 | Male | CD | BMS | 3,0x19mm | 45° OAE | 31,5kg | 33,5 | Minimal Hyperplasia |
| | | CX | DES | 3,5x19mm | | | | Without Hyperplasia |
| | | DA | DES | 3,5x19mm | | | | Without Hyperplasia |

There was a low mortality and restenosis. The results from patients in the bare metal stents (BMS) group were consistent with expected for metallic stents. The drug-free group indicates that the coating with biodegradable polymer does not cause inflammatory response. The coating with biodegradable polymer and sirolimus was the most effective in reducing neointimal hyperplasia.

Thus, the Solaris DE stent combines the advantages of Solaris stent and the advantages of drug-eluting stents (Inspiron^{®}) with a lower amount of drug, which is applied only on the ends of the stent. A clinical significance was observed in the image tests with a lower volume of neointimal hyperplasia after 60 days of use and a greater volume of lumen for Solaris DE when compared to the control Solaris (Table 8).

### Citation List

1. WO2010083530
2. US2016296351

## Claims

1. A drug-eluting stent (10) comprising:
- an expandable structural scaffold (20) configured to resist radial compression when disposed in a lumen of a patient, the structural scaffold comprising a central portion (21) arranged between a proximal end portion (22) and a distal end portion (23);
- a lining (30) arranged only at the central portion so that the central portion is covered by the lining and the end portions are free from the lining; wherein the lining comprises a biocompatible thermoplastic polymer and, optionally, a fibering agent;
- wherein the proximal and the distal end portions are coated with a drug-containing coating (40), the coating comprising a biodegradable polymer and a Limus drug; and
wherein the central portion of the structural scaffold and the lining are free from the Limus drug.

2. The drug-eluting stent according to claim 1, wherein the lining is drug-free.

3. The drug-eluting stent according to claims 1 or 2, wherein the structural scaffold (20) comprises a luminal surface (24) and an abluminal surface (25), and wherein the lining (30) comprises an inner lining layer (31) covering the luminal surface of the central portion of the structural scaffold and/or an outer lining layer (32) covering the abluminal surface of the central portion of the structural scaffold.

4. The drug-eluting stent according to any one of claims 1 to 3, wherein the biocompatible thermoplastic polymer is selected from the group consisting of polytetrafluoroethylene (PTFE), polyvinylidene fluoride (PVDF), polyurethane, and a mixture thereof.

5. The drug-eluting stent according to claim 4, wherein the biocompatible thermoplastic polymer is electrospun PTFE or expanded polytetrafluoroethylene (ePTFE).

6. The drug-eluting stent according to any one of claims 1 to 5, wherein the biodegradable polymer is a poly α-hydroxy acid; optionally, wherein the poly α-hydroxy acid is selected from the group consisting of poly-L-lactic acid (PLLA), poly-D-lactic acid (PDLA), poly-lactic acid, polyglycolic acid (PGA), poly (lactide-co-glycolic acid) (PLGA), and mixtures thereof.

7. The drug-eluting stent according to any one of claims 1 to 6, wherein the drug-containing coating (40) is arranged on the luminal surface, on the abluminal surface, or both, of the proximal (22) and the distal (23) end portions.

8. The drug-eluting stent according to any one of claims 1 to 7, wherein the Limus drug is selected from the group consisting of sirolimus (rapamycin), pimecrolimus, tacrolimus, everolimus, zotarolimus, novolimus, myolimus, temsirolimus, deforolimus, biolimus or combinations thereof

9. The drug-eluting stent according to any one of claims 1 to 8, wherein the Limus drug has a concentration from 1 to 10 µg/mm² of coated surface, in particular from 2 to 5 µg/mm² of coated surface.

10. The stent according to any one of claims 1 to 9, wherein the biodegradable polymer and the Limus drug are in a weigh ratio from 1.7:1 to 3.5:1 and, optionally, the drug-containing coating has a thickness of 20 µm.

11. The drug-eluting stent according to any one of claims 1 to 10, wherein the drug-containing coating (40) further comprises a second active agent, optionally, wherein the second active agent is an anticoagulant agent.

12. The drug-eluting stent according to claim 11, wherein the second active agent is heparin or a low molecular weight heparin (LMWH).

13. The drug-eluting stent according to any one of claims 1 to 12, wherein the structural scaffold (20) has a length from 40 to 100 mm and the drug-coated proximal (22) and distal (23) end portions have, independently, a length from 0.5 to 30 mm.

14. A method of manufacturing a drug-eluting stent (10), the method comprising:
(a) providing an expandable structural scaffold (20) configured to resist radial compression when disposed in a lumen of a patient, the structural scaffold comprising a central portion (21) arranged between a proximal end portion (22) and a distal end portion (23);
(b) covering only the central portion of the structural scaffold with a lining (30) of a biocompatible thermoplastic polymer so that the end portions are free from the lining, optionally, wherein the structural scaffold has a length from 40 to 100 mm and the drug-coated proximal and distal end portions have, independently, a length from 0.5 to 30 mm; and
(c) preparing a coating solution comprising a biodegradable polymer, a Limus drug, a solvent, and, optionally, a second active agent, and applying the coating solution to the proximal and distal end portions of the structural scaffold.

15. The method of claim 14, wherein step (b) is performed by electrospinning and the biocompatible thermoplastic polymer is PTFE.
